**Europäisches Patentamt**

(19) **European Patent Office**

**Office européen des brevets**

(11) Publication number : **0 400 888 B1**

(12) # EUROPEAN PATENT SPECIFICATION

(45) Date of publication of patent specification :
22.09.93 Bulletin 93/38

(51) Int. Cl.⁵ : **B01J 29/02**, B01J 27/053,
C07C 2/66

(21) Application number : **90305615.8**

(22) Date of filing : **23.05.90**

(54) **Alkylation process of aromatic compounds using a layered titanium oxide catalyst.**

(30) Priority : **30.05.89 US 358231**

(43) Date of publication of application :
**05.12.90 Bulletin 90/49**

(45) Publication of the grant of the patent :
**22.09.93 Bulletin 93/38**

(84) Designated Contracting States :
**BE DE FR GB IT NL**

(56) References cited :
**EP-A- 0 205 711**
**EP-A- 0 212 513**
**EP-A- 0 225 686**
**EP-A- 0 278 535**

(73) Proprietor : **MOBIL OIL CORPORATION**
**150 East 42nd Street**
**New York New York 10017 (US)**

(72) Inventor : **Aufdembrink, Brent Allen**
**10 Madelyn Avenue**
**Wilmington, Delaware 19803 (US)**
Inventor : **Le, Quang Ngoc**
**330 Rhode Island Avenue**
**Cherry Hill, New Jersey 08002 (US)**
Inventor : **Wong, Stephen Sui Fai**
**3 Carol Joy Road**
**Medford, New Jersey 08055 (US)**
Inventor : **Kresge, Charles Theodore**
**600 Thorncroft Drive**
**West Chester, Pennsylvania 19380 (US)**
Inventor : **Shim, Joosup**
**6 South Marion Avenue**
**Wenonah, New Jersey 08090 (US)**

(74) Representative : **Colmer, Stephen Gary et al**
**Patent Department c/o Mobil Services**
**Company Limited Mobil Court 3 Clements Inn**
**London WC2A 2EB (GB)**

## Description

This invention relates to layered titanium oxide catalysts.

Layered titanium oxides and their use as cracking and hydrocracking catalysts are disclosed in, for example, European Patent Publication Number EP-A-205711 and PCT International Publication Number WO 88/00090. It has now been found that sulfated layered titanium oxide catalysts are particularly effective as aromatics alkylation catalysts.

EP-A-212513 discloses a catalyst for removing nitrogen oxides from gases, which typically contain oxides of sulfur, comprising a layered silicate containing silica and titania between the layers.

The present invention resides in a process for preparing a long chain alkyl aromatic compound which comprises contacting at least one alkylatable aromatic compound with at least one alkylating agent possessing an alkylating aliphatic group having at least six carbon atoms in the presence of an alkylation catalyst comprising a layered material comprising a layered metal oxide and pillars of an oxide of at least one element selected from Groups IB, IIB, IIIA, IIIB, IVA, IVB, VA, VB, VIA, VIIA and VIIIA of the Periodic Table of the Elements separating the layers of the metal oxide, wherein each layer of the metal oxide comprises titanium oxide, said layered material further comprises sulfate ions, and said catalyst comprises at least 0.001 percent by weight of sulfur as measured by elemental analysis.

The present invention provides an advantageous process for alkylating aromatic compounds with relatively long chain alkylating agents to produce long chain alkyl aromatic products useful, inter alia, as intermediates in the manufacture of synthetic detergents. The alkylation of an aromatic hydrocarbon stream, e.g., one containing one or more of benzene, toluene, xylene and naphthalene, with a relatively long chain alkylating agent may also produce an aromatic lube base stock of low pour and cloud point, high viscosity and improved thermal and oxidative stability properties.

The term "aromatic" in reference to the alkylatable compounds which are useful herein is to be understood in accordance with its art-recognized scope which includes alkyl substituted and unsubstituted mono- and polynuclear compounds. Compounds of an aromatic character which possess a hetero atom are also useful provided they do not act as catalyst poisons under the reaction conditions selected.

Substituted aromatic compounds which can be alkylated herein must possess at least one hydrogen atom directly bonded to the aromatic nucleus. The aromatic rings can be substituted with one or more alkyl, aryl, alkaryl, alkoxy, aryloxy, cycloalkyl, halide, and/or other groups which do not interfere with the alkylation reaction.

Suitable aromatic hydrocarbons include benzene, naphthalene, anthracene, naphthacene, perylene, coronene and phenanthrene.

Generally, the alkyl groups which can be present as substituents on the aromatic compound contain from one to 40 carbon atoms, e.g., from one to eight carbon atoms, and more typically from about one to four carbon atoms.

Suitable alkyl substituted aromatic compounds include toluene, xylene, isopropylbenzene, normal propylbenzene, alpha-methylnaphthalene, ethylbenzene, cumene, mesitylene, durene, p-cymene, butylbenzene, pseudocumene, o-diethylbenzene, m-diethylbenzene, p-diethylbenzene, isoamylbenzene, isohexylbenzene, pentaethylbenzene, pentamethylbenzene; 1,2, 3, 4-tetraethylbenzene; 1,2,3,5-tetramethylbenzene; 1,2,4-triethylbenzene, 1,2,3-trimethylbenzene, m-butyltoluene; p-butyltoluene; 3,5-diethyltoluene; o-ethyltoluene; p-ethyltoluene; m-propyltoluene; 4-ethyl-m-xylene; dimethylnaphthalenes; ethylnaphthalene; 2,3-dimethylanthracene; 9-ethylanthracene; 2-methylanthracene; o-methylanthracene; 9,10-dimethylphenanthrene; and 3-methylphenanthrene. Higher molecular weight alkylaromatic hydrocarbons can also be used as starting materials and include aromatic hydrocarbons such as are produced by the alkylation of aromatic hydrocarbons with olefin oligomers. Such products are frequently referred to in the art as alkylate and include hexylbenzene, nonylbenzene, dodecylbenzene, pentadecylbenzene, hexyltoluene, nonyltoluene, dodecyltoluene and pentadecyltoluene. Very often alkylate is obtained as a high boiling fraction in which the alkyl group attached to the aromatic nucleus varies in size from $C_6$ to $C_{20}$.

Reformate containing substantial quantities of benzene, toluene and/or xylene constitutes a particularly useful feed for the alkylation process of this application.

The alkylating agents which are useful in the process of this application generally include any aliphatic or aromatic organic compound having one or more available alkylating aliphatic groups capable of reaction with the alkylatable aromatic compound. The alkylatable group itself should have at least 6, preferably at least 8, and more preferably at least 10, carbon atoms. Examples of suitable alkylating agents are olefins such as hexenes, heptenes, octenes, nonenes, decenes, undecenes and dodecenes; alcohols (inclusive of monoalcohols, dialcohols and trialcohols) such as hexanols, heptanols, octanols, nonanols, decanols, undecanols and dodecanols; and alkyl halides such as hexyl chlorides, octyl chlorides, dodecyl chlorides; and, higher homologs of the foregoing. Branched alkylating agents, especially oligomerized olefins such as the trimers, tetramers and pentamers, of light olefins such as ethylene, propylene, and butylene, or of heavier olefins such as octene, decene and dodecene, are also useful herein.

The sulfated catalysts described herein are prepared from layered starting materials. These layered starting materials comprise titanium oxide in the metal oxide layers thereof. These metal oxide layers are negatively charged, the negative charges thereof being balanced by interlayer cations. It will be understood that the term, titanium oxide, as used herein, especially in reference to metal oxides in the metal oxide layers, is intended to connote negatively charged forms of titanium oxide such as titanates.

The final layered catalysts may be prepared by a method which comprises the steps of starting with said layered metal oxide and physically separating the layers thereof by introducing an organic cationic species between the layers at interlayer anionic sites associated with the layered oxide, introducing between the separated layers of the layered oxide a compound capable of conversion to an oxide and then converting said compound to the oxide to form oxide pillars separating adjacent layers of the layered oxide.

It is to be appreciated that the term "layered" metal oxide is used herein in its commonly accepted sense to refer to a material which comprises a plurality of separate metal oxide layers which are capable of being physically displaced away from one another such that the spacing between adjacent layers is increased. Such displacement can be measured by X-ray diffraction techniques and/or by density measurements.

These pillared oxide products may have relatively high interplanar distances (d-spacings), e.g., greater than 6 Angstrom, more typically greater than 10 Angstrom, and even greater than 20 Angstrom. These materials are capable of being exposed to severe conditions such as those encountered in calcining, e.g., at temperatures of 450°C for two or more hours, e.g., four hours, in nitrogen or air, without significant decrease, e.g., less than 10%, in interlayer distance. Furthermore, such pillared oxides can be prepared without the severe dilution often necessary to introduce the interspathic material in prior art techniques of interlayering. Also, the size of interspathic oxide contained within the final product can be greatly varied because the oxide precursor species may be introduced in an electrically neutral form such that the amount of interspathic material incorporated within the layered titanometallate is not dependent upon the charge density of the original layered oxide.

Certain of the layered starting materials, which are used to prepare sulfated catalysts, are described in PCT International Publication Number WO 88/00090, published January 14, 1988. These layered materials comprise a layered metal oxide and pillars of an oxide of at least one element selected from Groups IB, IIB, IIIA, IIIB, IVA, IVB, VA, VB, VIA, VIIA and VIIIA of the Periodic Table of the Elements (Fisher Scientific Co. Cat. No. 5-702-10, 1978) separating the layers of the metal oxide, wherein each layer of the metal oxide has the general formula

$$[M_x\square_yZ_{2-(x+y)}O_4]^{q-}$$

wherein M is at least one metal of valence n wherein n is an integer between 0 and 7 and preferably is 2 or 3, $\square$ represents a vacancy site, Z is a tetravalent metal, preferably titanium, and wherein

$$q = 4y - x(n-4)$$

and preferably is 0.6-0.9,

$$O < x + y < 2$$

Interposed between the negatively-charged layers of the oxide will be charge-balancing cations A of charge m wherein m is an integer between 1 and 3, preferably 1. Preferably A is a large alkali metal cation selected from the group consisting of Cs, Rb and K and M is a divalent or trivalent metal cation selected from at least one of Mg, Sc, Mn, Fe, Cr, Ni, Cu, Zn, In, Ga and Al. For example, M can be both In and Ga. Structurally, these metal oxides consist of layers of $(M_x\square_yZ_{1-x-y})O_6$ octahedra which are _trans_ edge-shared in one dimension and _cis_ edge-shared in the second dimension forming double octahedral layers which are separated by the A cations in the third dimension. When Z is titanium, these materials can be prepared by high temperature fusion of a mixture of 1) metal oxide, 2) alkali metal carbonate or nitrate and 3) titanium dioxide; or by fusion of a mixture of alkali metallate and titanium dioxide. Such fusion can be carried out in air in ceramic crucibles at temperatures ranging between 600 to 1100°C after the reagents have been ground to an homogeneous mixture. The resulting product is ground to 20 to 250 mesh, preferably about 100 mesh, prior to the organic swelling and polymeric oxide intercalation steps.

Further description of layered titanometallate starting materials and their methods of preparation can be found in the following references:

Reid, A.F.; Mumme, W.G.; Wadsley, A.D. Acta Cryst. (1968), B24, 1228; Groult, D.; Mercy, C.; Raveau, B. J. Solid State Chem. 1980, 32 289; England, W.A.; Burkett, J.E.; Goodenough; J.B., Wiseman, P. J. J. Solid State Chem. 1983, 49 300.

Use of these layered metal oxides as a layered starting material permits inclusion of different metal atoms into the layered starting material being treated which allows potential catalytically active sites to be incorporated in the stable layer itself. Moreover, variable amounts of metal atoms may be added to provide a catalyst with optimum activity for a particular process. Furthermore, the infinite trans-edge shared layer structure of the titanometallates instead of the sheared 3-block structure of, for example, $Na_2Ti_3O_7$ may reduce or eliminate shearing of the layers as a possible mechanism for thermal or hydrothermal decomposition of the calcined intercalated material. The variable charge density on the oxide layer, possible for these layered metal oxides due to the various oxidation states of metal oxides, the incorporated metal atom and the varying stoichiometry of the materials, may allow variation in the amount of the organic cationic species which can be exchanged into the material. This variation, in turn, permits variation of the ultimate concentration of the oxide pillars between the layers of the final product.

The pillared layered materials which are sulfated may also be pillared titanate compounds. Pillared trititanate compounds are described in published European Patent Publication No. 205711, as well as in U.S. Patent No. 4,600,503. Trititanates are commercially available materials and consist of anionic sheets of titanium octahedra with interlayer alkali metal cations which can be exchanged for interspathic $H^+$ and $H_3O^+$ ions. A method for making such material may be found in U.S. Patent 2,496,993. It is known that the interlayer distance of $Na_2Ti_3O_7$ may be increased by replacing interlayer sodium ions with larger octylammonium ions. See, Weiss et al., Angew. Chem/72 Jahrg. 1960/Nr/2, pp 413-415. However, the organic-containing trititanate is highly susceptible to heat which can remove the organic material and cause collapse of the layered structure. The insertion of stable oxide pillars between adjoining layers results in a heat-stable material which substantially retains its interlayer distance upon calcination.

To produce the pillared catalyst substrate of the present invention, the layered metal oxide starting material may be initially treated with a "propping" agent comprising a source of organic cation, such as organoammonium cation, in order to effect an exchange of, or addition to, the interspathic cations resulting in the layers of the starting material being propped apart. Suitable organoammonium cations include C3 and higher alkylammonium cations such as n-dodecylammonium, octylammonium, n-heptylammonium, n-hexylammonium and n-propylammonium. The extent of the interlayer spacing can be controlled by the size of the organoammonium ion employed so that use of the n-propylammonium cation can achieve a interlayer spacing of 2 to 5 Angstrom whereas to achieve an interlayer spacing of 10 to 20 Angstrom an n-octylammonium cation or a cation of equivalent length is required. Indeed, the size and shape of the organic cation can affect whether or not it can be incorporated within the layered structure at all. For example, bulky cations such as tetrapropylammonium are generally undesirable for use while n-alkyl ammonium cations such as those derived from n-alkyl primary amines and $R_3R'N^+$ cations where R is methyl or ethyl and R is an n-alkyl group with at least 5 carbon atoms, are preferred. Preferably treatment with the organic cationic species is conducted in aqueous media so that water is then available to hydrolyze the electrically neutral, hydrolyzable polymeric oxide precursor subsequently introduced into the "propped" product.

During this propping or swelling step it is desirable to maintain a low hydrogen ion concentration to prevent decomposition of the layered oxide structure as well as to prevent preferential sorption of hydrogen ion over the propping agent. A pH range of 6 to 10, preferably 7 to 8.5 is generally employed during treatment with the propping agent. After this treatment, it has been found advantageous to wash out excess propping agent using a suitable solvent followed by washing with water. For example, ethanol is a solvent for use with an n-octylamine propping agent. Such washing permits greater incorporation of the oxide pillar precursor in the layered metal oxide. The water treatment allows penetration of water into the interlayer spaces which assists in subsequent hydrolysis the oxide pillar precursor.

The foregoing treatment results in the formation of a layered metal oxide of enhanced interlayer separation depending upon the size of the organic cation introduced. In one embodiment, a series of organic cation exchanges can be carried out. For example, an organic cation may be exchanged with an organic cation of greater size, thus increasing the interlayer separation in a step-wise fashion. Preferably, contact of the layered oxide with the propping agent is conducted in aqueous medium so that water is trapped between the layers of the "propped" species.

It is preferred that the organic cation deposited between the layers be capable of being removed from the pillared material without substantial disturbance or removal of the interspathic polymeric oxide. For example, organic cations such as n-octylammonium may be removed by exposure to elevated temperatures, e.g., calcination, in nitrogen or air, or by chemical oxidation preferably after the interspathic polymeric oxide precursor has been converted to the polymeric oxide pillars in order to form the layered product.

Interspathic oxide pillars are then formed between the layers of the propped or swollen metal oxide starting material and may include an oxide, preferably a polymeric oxide, of zirconium or titanium or more preferably of an element selected from Group IVB of the Periodic Table (Fischer Scientific Company Cat. No. 5-702-10, 1978), other than carbon, i.e., silicon, germanium, tin and lead. Other suitable oxides include those of Group VA, e.g., V, Nb, and Ta, those of Group IIA, e.g., Mg or those of Group IIIB, e.g., B. Most preferably, the pillars include polymeric silica. In addition, the oxide pillars may include an element which provides catalytically active acid sites in the pillars, preferably aluminum.

The oxide pillars are formed from a precursor material which is preferably introduced between the layers of the organic "propped" species as a cationic, or more preferably, electrically neutral, hydrolyzable compound of the desired elements, e.g., those of Group IVB. The precursor material is preferably an organometallic compound which is a liquid under ambient conditions. In particular, hydrolyzable compounds, e.g., alkoxides, of the desired elements of the pillars are utilized as the precursors. Suitable polymeric silica precursor materials include tetraalkylsilicates, e.g., tetrapropylorthosilicate, tetramethylorthosilicate and, most preferably, tetraethylorthosilicate. Where the pillars also include polymeric alumina, a hydrolyzable aluminum compound can be contacted with the organic "propped" species before, after or simultaneously with the contacting of the propped titanometallate with the silicon compound. Preferably, the hydrolyzable aluminum compound employed is an aluminum alkoxide, e.g., aluminum isopropoxide. If the pillars are to include titania, a hydrolyzable titanium compound such as titanium alkoxide, e.g., titanium isopropoxide, may be used. In addition, the oxide precursor may contain zeolite precursors such that exposure to conversion conditions results in the formation of interspathic zeolite material as at least part of the oxide pillars.

After hydrolysis to produce the oxide pillars and calcination to remove the organic propping agent, the final pillared product may contain residual exchangeable cations. Such residual cations in the layered material can be ion exchanged by known methods with other cationic species to provide or alter the catalytic activity of the pillared product. Suitable replacement cations include cesium, cerium, cobalt, nickel, copper, zinc, manganese, platinum, lanthanum, aluminum, ammonium, hydronium and mixtures thereof.

The resulting pillared products exhibit thermal stability at temperatures of 500°C or even higher as well as substantial sorption capacities (as much as 10 to 25 wt% for $H_2O$ and $C_6$ hydrocarbon). Silica-pillared products possess interlayer separations of greater than 12A and surface areas greater than 250 $m^2/g$ when divalent metal atoms, e.g., Mg, Ni, Cu and Zn, are present as the metal M of the product. Silica-pillared products incorporating trivalent metal atoms, e.g., Sc, Mn, Fe, Cr, In, Ga and Al can possess interlayer separations of 6 to 15 Å.

The layered material alkylation catalyst described herein can optionally be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be exchanged into the composition, impregnated therein or intimately physically admixed therewith. Such component can be impregnated in, or on, the layered material such as, for example, by, in the case of platinum, treating the layered material with a solution containing a platinum metal-containing ion. Thus, suitable platinum compounds for this purpose include chloroplatinic acid, platinous chloride and various compounds containing the platinum amine complex.

The layered material may be subjected to thermal treatment, e.g., to decompose organoammonium ions. This thermal treatment is generally performed by heating one of these forms at a temperature of at least 370°C for at least 1 minute and generally not longer than 20 hours. While subatmospheric pressure can be employed for the thermal treatment, atmospheric pressure is preferred simply for reasons of convenience. The thermal treatment can be performed at a temperature of up to 925°C.

Prior to its use in the alkylation process described herein, the layered material catalyst should be dehydrated, at least partially. This dehydration can be done by heating the crystals to a temperature in the range of from 200°C to 595°C in an atmosphere such as air, nitrogen, etc., and at atmospheric, subatmospheric or superatmospheric pressures for between 30 minutes to 48 hours. Dehydration can also be performed at room temperature merely by placing the layered material in a vacuum, but a longer time is required to obtain a sufficient amount of dehydration.

It may be desirable to incorporate the layered material with another material which is resistant to the temperatures and other conditions employed in the alkylation process described herein. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with layered material, i.e., combined therewith or present during its synthesis, which itself is catalytically active may change the conversion and/or selectivity of the catalyst. Inactive materials suitably serve as diluents to control the amount of conversion so that alkylation products can be obtained economically and orderly without employing other means for controlling the rate of reaction. These materials may be incorporated into naturally occurring clays, e.g., bentonite and kaolin, to improve the crush strength of the catalyst under commercial alkylation operating conditions. Said materials, i.e., clays, oxides, etc., function as binders for the catalyst. It is desirable to provide a catalyst having good crush strength because in commercial use, it is desirable to prevent the catalyst from breaking down into powder-like materials. These clay binders have been employed normally only for the purpose of improving the crush strength of the catalyst.

Naturally occurring clays which can be composited with layered materials include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with layered materials also include inorganic oxides, notably alumina.

In addition to the foregoing materials, the layered materials can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia.

The relative proportions of finely divided layered materials and inorganic oxide matrix vary widely, with the layered material content ranging from about 1 to 90 percent by weight and more usually, particularly when the composite is prepared in the form of beads, in the range of 2 to 80 weight of the composite.

According to the present invention, the alkylation activity of the layered catalyst is increased by including sulfate ions therein. The effect of sulfate promotion on the generation of strong acidity on metal oxides such as $ZrO_2$, $TiO_2$ and $Fe_2O_3$, has been observed by K. Tanabe as reported in "Heterogenous Catalysis", IUCCP Meeting at Texas A & M, April 1-4, 1984, B. L. Shapiro (Editor), pp. 71-94, and by T. Jin, T. Yamaguchi and K. Tanabe as reported in J. Phys. Chem., 90, 4794-4796, 1986.

A preferred method for incorporating sulfate ions into the layered materials involves contacting the materials with a source of sulfate ions, such as sulfuric acid. Aqueous solutions of sulfate ions, such as aqueous solutions of sulfuric acid or ammonium sulfate, may also be used as sources of sulfate ions.

The layered materials may be contacted with the aqueous solutions of sulfate ions under ambient conditions of temperature and pressure. These conditions of temperature and pressure may vary. As pointed out in the aforementioned article by Tanabe, it is believed that sulfate ions form a complex with titanium. Accordingly, contacting conditions should be selected so as to form this complex. Although the association of sulfate ions with titanium is believed to be strong enough to withstand washing with water, such a washing step is not necessary, especially when aqueous solutions of sulfuric acid or ammonium sulfate are used as sulfate ion sources. After contact with an aqueous solution of sulfate ions, the sulfate containing layered materials may be recovered by decanting the mother liquor, e.g. by filtration, or by evaporating the mixture of layered material and aqueous sulfate solution to dryness. Wet sulfate-containing layered material may be dried by calcination procedures described previously herein, e.g., from temperatures ranging from 200 to 595°C.

Sulfate ions may also be incorporated into the present titanium-containing materials by contacting these materials with $SO_3$. The $SO_3$ may be converted to sulfate ions, in situ, by moisture already contained in the layered material or by water, e.g., water vapor or steam, applied after contact with $SO_3$.

The layered material may also be contacted with other compounds, such as $H_2S$ or $SO_2$, which are capable of being converted to sulfate ions by oxidation. For example, the titanium-containing layered material may be contacted with $H_2S$ or $SO_2$ along with or followed by oxidation in a source of oxygen, such as air, to convert sulfur to the plus 6 valance state.

The amount of sulfate ions incorporated into the layered materials should be sufficient to provide at least 0.001 percent by weight, conveniently from 0.1 to 10 percent by weight, more preferably from 0.5 to 10 percent by weight, and most preferably from 1 to 3 percent by weight, of sulfur as measured by elemental analysis of the catalyst, based upon the entire weight of the catalyst.

The alkylation process described herein may be conducted under conditions including a temperature of 0 to 500°C, a pressure of from 20 to 25000 kPa (0.2 to 250 atmospheres), a feed weight hourly space velocity (WHSV) of 0.1 to 500 and an alkylatable aromatic compound to alkylating agent mole ratio of 0.1:1 to 50:1. The WHSV is based upon the weight of the catalyst composition employed, i.e., the total weight of active catalyst (and binder if present). Preferred reaction conditions include a temperature of 100 to 350°C, a pressure of 100 to 2500kPa (1 to 25 atmospheres), a WHSV of 0.5 to 100 and an alkylatable aromatic compound to alkylating agent mole ratio of 0.5:1 to 5:1. The reactants can be in either the vapor phase or the liquid phase and can be neat, i.e., free from intentional admixture or dilution with other material, or they can be brought into contact with the layered material catalyst composition with the aid of carrier gases or diluents such as, for example, hydrogen or nitrogen.

The alkylation process described herein can be carried out as a batch-type, semi-continuous or continuous operation utilizing a fixed or moving bed catalyst system. A preferred embodiment entails use of a catalyst zone wherein the hydrocarbon charge is passed concurrently or countercurrently through a moving bed of particle-form catalyst. The latter, after use, is conducted to a regeneration zone where coke is burned from the catalyst in an oxygen-containing atmosphere (such as air) at elevated temperature, after which the regenerated catalyst is recycled to the conversion zone for further contact with the organic reactants.

In the Examples which follow, whenever sorption data are set forth for comparison of sorptive capacities for water, cyclohexane and/or n-hexane, they were equilibrium adsorption values determined as follows:

A weighed sample of the calcined adsorbent was contacted with the desired pure adsorbate vapor in an adsorption chamber, evacuated to less than 1 mm and contacted with 2.8 kPa (21 Torr) of water vapor and 5.3 kPa (40 Torr) of n-hexane or cyclohexane vapor, pressures less than the vapor-liquid equilibrium pressure of the respective adsorbate at 90°C. The pressure was kept constant (within about ± 0.5 mm) by addition of adsorbate vapor controlled by a manostat during the adsorption period, which did not exceed about 8 hours. As adsorbate was adsorbed by the layered material, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 of calcined adsorbant.

Example 1

This Example describes the preparation of a layered catalyst. $Cs_2CO_3$ (621g) and $TiO_2$ (795g) were fired at 650°C three times, with intermediate grindings between firings. The fired material was ball-milled for 4 hrs (30% solids in $H_2O$), then exchanged five times with 1 M $NH_4NO_3$ (10 ml $NH_4NO_3$/g solid) at reflux for 20 hrs. After each exchange the sample was filtered and washed with 2 1 hot water. The ammonium exchanged solid was swollen by refluxing in neat octylamine for 24 hrs. using a Dean-Stark trap in the condensation column to remove water from the system. The swollen solid was filtered and washed with 2000 ml EtOH, then air dried. This solid was treated with tetraethylorthosilicate (5g TEOS/g solid) under nitrogen at 80°C for 20 hrs, filtered and dried under nitrogen. The TEOS treatment was repeated once. The pillared material was obtained by calcining the dried TEOS treated material in flowing air at 500°C for 240 minutes. Chemical and physical properties of the catalyst are summarized in the following table:

```
Cs, ppm                         23
Ti, wt%                       29.0
SiO₂, wt%                     44.9

Ash, wt% (1000°C)           97.52
Surface Area, m²/g            526

Sorption, wt%
  H₂O                        22.7
  n-C₆                       14.3
  cyclohexane                15.7

Density, g/cc
  Real                      2.799
  Particle                   0.83
```

Example 2

This Example illustrates the catalytic activity of the catalyst prepared from Example 1 for alkylating benzene with the long chain alpha olefin, alpha tetradecene, a C-14 olefin. The alkylation reaction was carried out in a 1 liter autoclave. The conditions were 250 g of alpha C-14 olefin (Shell Neodene-14) and 50g of benzene (2:1 mole ratio of olefin and benzene) with 23.7g of Example 1 catalyst at 200°C (400°F) for 6 hours under nitrogen pressure of 2860 kPa (400 psig). After decanting and filtering the catalyst, the total liquid product was then vacuum distilled at 343°C (650°F) to obtain a 63 wt% lube range material with the following properties:

```
Pour point, °F(°C)     -50 (-45)
Cloud point, °F(°C)    -44 (-42)

KV @40°C, cSt          21.41
KV @100°C, cSt         4.521

VI                       127
```

Example 3

This Example describes sulfate treatment of the Example 1 catalyst. The Example 1 catalyst (20 g) was stirred in 500 ml of 1 N $H_2SO_4$ for 10 minutes, filtered and air dried to yield 22.39 g of sulfate-containing material. The solid was dried at 120°C for 2 hrs. (22.3 g yield), then calcined at 500°C for 60 minutes to produce 19.0 g of sulfated treated catalyst.

Example 4

Under experimental conditions identical to those used in Example 2, sulfate-containing catalyst prepared in Example 3 is more active for the alkylation reaction and provides about 92 wt% lube yield as compared to 63 wt% without sulfate treatment. The properties of alkylated benzene lubes produced from the layered material catalyst with and without sulfate promotion are shown as follows:

|  | Example 2 | Example 4 |
|---|---|---|
| Catalyst | Ex. 1 | Ex. 3 |
| Sulfate Treatment | No | Yes |
| S on Catalyst, wt% | -- | 1.22 |
| Surface Area, $m^2$/g | 526 | 327 |
| Lube Yield, wt% | 63 | 92 |
| **Lube Properties** | | |
| Pour point, °F(°C) | -50 (-45) | -50 (-45) |
| Cloud point, °F(°C) | -44 (-42) | -50 (-45) |
| KV @40°C, cSt | 21.41 | 23.70 |
| KV @100°C, cSt | 4.521 | 4.826 |
| VI | 127 | 128 |

Analysis indicated these synthetic lubricants to comprise mainly a mixture of mono-, di- and tri-alkyl benzenes with a C-14 alkyl chain length. To much lesser extent, the layered material catalysts also promote oligomerization reactions under these process conditions. Lowering the olefin:benzene ratio to less than 2 would eliminate or reduce the oligomerization reaction. These alkylbenzene lubricants have excellent low temperature viscometric properties as indicated by very low pour and cloud points (< -45°C). They may find wide temperature range applications because of their high VI of 127-128.

Example 5

This Example describes another sulfate treatment of the Example 1 catalyst. The Example 1 catalyst (50.0g) was stirred in 1000 ml 1 M $(NH_4)_2(SO_4)$ for 15 minutes, filtered, and air dried. The solid was dried at 120°C for 7 h, then calcined at 500°C for 60 minutes to produce 37.98 g of sulfate treated catalyst. Chemical and physical analyses indicated the catalyst had the following properties:

19.3 wt % Ti
48.2 wt % $SiO_2$
3.180 wt % S
89.35 wt % Ash (1000°C)
226 $m^2$/g Surface Area
Sorptions 11.6% $H_2O$, 6.4% c-$C_6$, 5.6% n-$C_6$

Example 6

This Example describes another sulfate treatment of the Example 1 catalyst. The Example 1 catalyst (50.0g) was impregnated with 43.0 g 1 M $H_2SO_4$ for 15 minutes, filtered, and air dried. The solid was dried at 120°C for 7 h, then calcined at 500°C for 60 minutes to produce 37.98 g of sulfate treated catalyst.

Chemical and physical analyses indicated the catalyst had the following properties:

20.2 wt % Ti
55.7 wt % $SiO_2$
1.910 wt % S
90.63 wt % Ash (1000°C)
283 $m^2$/g Surface Area
Sorptions 13.5% $H_2O$, 3.2% c-$C_6$, 7.2% n-$C_6$

Example 7

This Example describes another sulfate treatment of the Example 1 catalyst. The Example 1 catalyst (50.0g) was impregnated with 43.0 g 1 M $(NH_4)_2(SO_4)$. The solid was then air dried. The solid was dried at 120°C for 7 h, then calcined at 500°C for 60 minutes to produce 39.90 g of sulfate treated catalyst.

Chemical and physical analyses indicated the catalyst had the following properties:

19.0 wt % Ti
51.5 wt % $SiO_2$
2.080 wt % S
90.97 wt % Ash (1000°C)
356 $m^2$/g Surface Area
Sorptions 17.1% $H_2O$, 9.3% c-$C_6$, 8.9% n-$C_6$

Example 8

This Example describes the preparation of a pillared trititanate material. All treatments in the following procedure were carried out at room temperature unless otherwise specified.

A sample of $Na_2Ti_3O_7$ was prepared by calcining an intimate mixture of 1000 g of $TiO_2$ and 553 g $Na_2CO_3$ in air at 1000°C for 20 hours (heat up rate = 3°C/min). This mixture was then reground and reheated in air at 1000°C for 20 hours (heat up rate = 3°C/min). The product was stirred in 1.5 liters of water for 1 hours, filtered, dried in air at 121°C for one hour, and calcined in air at 540°C for one hour. This product was slurried in 900 grams of water and ballmilled for 16 hours. The solid $Na_2Ti_3O_7$ product was filtered and dried for 24 hours.

The entire 1,194 gram batch of this ballmilled product was exchanged five times at 85-91°C with a solution of 4,240 grams of 50% ammonium nitrate diluted to 10 liters with water. The solid product was filtered and washed with 20 liters of water after each exchange. This product was dried for 24 hours after the final exchange.

An 800 gram batch of the ammonium-exchanged trititanate was refluxed with stirring for 48 hours in a mixture of 800 grams of octylamine in 5,300 grams of water. Subsequently, another 800 grams aliquot of octylamine was added, and the resulting mixture was stirred at reflux for an additional 6 days and then at room temperature for 7 additional days. The solution was decanted and the solids filtered using one liter of ethanol to assist filtration. The product was washed with 20 liters of water and dried overnight. Seven hundred and fifty grams of this product was refluxed with 1,000 grams of octylamine for six hours. The mixture was cooled to less than 70°C (160°F), one liter of ethanol was added, and the product was filtered and dried overnight.

This dried material was reslurried in one liter of ethanol for one hour, filtered, and dried for 24 hours. The product was then refluxed in 750 grams of octylamine in a four-necked, five liter round bottom flash equipped with a Dean-Stark trap. Reaction temperature increased in a one hour interval from 135 to 175°C (275 to 347°C) and water was removed from the system via the Dean-Stark trap. The mixture was refluxed at 175°C (347°F) for two hours and then cooled to less than 70°C (160°F). One liter of ethanol was then added, and the solid product was filtered and dried overnight. The dried product was reslurried in one liter of ethanol for one hour, filtered, and dried again overnight. This product was then stirred in three liters of water for 24 hours, filtered, and dried for 42 hours. The octylammonium swollen trititanate had the following composition:

$TiO_2$ 73.1 wt%
C 8.97 wt%
N 2.3 wt%
Na 1160 ppm
Ash 77.4 wt%

Four hundred grams of the octylammonium swollen trititanate from above was mechanically stirred with 2,700 grams of tetraethylorthosilicate in a four-necked, five liter round bottom flask equipped with nitrogen inlet and outlet lines. This mixture was stirred under a slow nitrogen purge for three days. The product was filtered and dried for 24 hours. This material was then stirred in two liters of water for 24 hours, filtered, and dried for 48 hours. The tetraethylorthosilicate treatment was repeated as described above. The product of this step, after filtration and drying, was stirred in two liters of water for 24 hours. This product was filtered, dried for 24 hours, and calcined at 510°C (950°F) in nitrogen for two hours at a heat-up rate of 3°C (5°/min. The atmosphere was then changed to air and the material was calcined for an additional one hour at 510°C (950°F). The final low sodium silicotitanate had an alpha value of 1.6 and the composition and physical properties shown below:

| | |
|---|---|
| Surface Area, $m^2$/g | 292 |
| Sorption, g/100g | |
| n-hexane | 8.3 |
| cyclohexane | 9.2 |
| water | 12.3 |
| | |
| Composition, wt% | |
| $TiO_2$ | 73.9 |
| $SiO_2$ | 22.7 |
| Na | 0.114 |
| Ash | 97.4 |

Example 9

This Example describes the sulfate treatment of the silicotitanate material from Example 8. 20.0 grams of the silicotitanate material from Example 8 was stirred in 500 ml 1N $H_2SO_4$ for 10 minutes, filtered and air dried to yield 19.9 g of sulfated-containing material. The solid was dried at 120°C for 2 hours (19.1 g), then calcined at 500°C for 1 hr to produce 17.8 g of sulfate treated product.

Example 10

This Example illustrates the catalytic activity of sulfate-treated silicotitanate prepared from Example 9 for alkylating benzene with decene oligomers. A mixture of decene oligomers (33 wt.% $C_{30}$, 52 wt% $C_{40}$ and 15 wt% $C_{50}$) was obtained from oligomerization of 1-decene over $BF_3$-propanol catalyst system according to U.S. 3,769,363. The subsequent alkylation reaction was carried out using 250 g of decene oligomers and 78 g of benzene with 16 g of sulfate-containing Example 9 catalyst. The reaction conditions included a reaction temperature of about 200°C (400°F). The reaction took place for about 6 hours under nitrogen pressure of about 2860 kPa (400 psig). The total liquid product was then distilled at 343°C (650°F) to remove any unconverted benzene. IR analysis of 343°C+ (650°F+) product indicated that there is a significant amount of benzene incorporated into the backbone of decene oligomers. The product properties and quality of decene oligomers before and after alkylating with benzene are shown as follows:

| PROCESS | OLIGOMERIZATION | ALKYLATION |
|---|---|---|
| Catalyst | $BF_3$-propanol | Example 9 |
| **Product Properties** | | |
| Pour Point, °F(°C) | -65 (-54) | -65 (-54) |
| Cloud Point, °F(°C) | -65 (-54) | -65 (-54) |
| KV @40°C, cSt | 25.73 | 27.67 |
| KV @100°C, cSt | 5.225 | 5.449 |
| VI | 138 | 137 |
| **Product Quality** | | |
| Thermal Stability @ 288°C (550°F) % Viscosity Decrease | 16.4 | 5.6 |
| B-10 Oxidation @ 127°C (260°F) 40 hrs. % Viscosity Increase | 120 | 92 |
| DSC-IP @180°C, minutes | 5.0 | 7.5 |

The above results indicate that the alkylation reaction catalyzed by the sulfate-containing silicotitanate material produces a benzene-containing synthetic lube base stock with excellent product properties (very low pour and cloud point, high VI) and improved thermal and oxidative stability.

## Claims

1. A process for preparing a long chain alkyl aromatic compound which comprises contacting at least one alkylatable aromatic compound with at least one alkylating agent possessing an alkylating aliphatic group having at least six carbon atoms in the presence of an alkylation catalyst comprising a layered material comprising a layered metal oxide and pillars of an oxide of at least one element selected from Groups IB, IIB, IIIA, IIIB, IVA, IVB, VA, VB, VIA, VIIA and VIIIA of the periodic Table of the Elements separating the layers of the metal oxide, wherein each layer of the metal oxide comprises titanium oxide, said layered material further comprises sulfate ions, and said catalyst comprises at least 0.001 percent by weight of sulfur as measured by elemental analysis.

2. A process as claimed in claim 1 wherein the catalyst comprises 0.1 to 10% by weight of sulfur as measured by elemental analysis.

3. A process as claimed in claim 1 wherein the catalyst comprises 1 to 3% by weight of sulfur as measured by elemental analysis.

4. A process as claimed in claim 1 wherein each layer of the layered metal oxide has the general formula
$$[M_x\square_yZ_{2 - (x + y)}O_4]^{q-}$$
wherein M is at least one metal of valence n wherein n is an integer between 0 and 7, $\square$ represents a vacancy site, Z is titanium, and wherein
$$q = 4y - x(n - 4)$$
$$0 < x + y < 2$$

5. A process as claimed in claim 1 wherein said lead material comprises trititanate layers.

6. A process as claimed in claim 1 wherein the pillars comprise polymeric silica.

7. A process as claimed in claim 1 wherein said sulfate ions are produced by pretreating the catalyst with a aqueous solution of sulfuric acid.

8. A process according to claim 1, wherein alkylation is effected at a temperature of 0 to 500°C, a pressure of 20 to 25000 kPa (0.2 to 250 atmospheres), a WHSV of 0.1 to 500 and an alkylatable aromatic compound to alkylating agent mole ratio of 0.1:1 to 50:1.

9. A process as claimed in claim 1, wherein alkylation is effected at a temperature of 100 to 350°C, a pressure of 100 to 2500 kPa (1 to 25 atmospheres), a WHSV of 0.5 to 100 and an alkylatable aromatic compound to alkylating agent mole ratio of 0.5:1 to 5:1.

## Patentansprüche

1. Verfahren zur Herstellung einer langkettigen alkylaromatischen Verbindung, das den Kontakt von zumindest einer alkylierbaren aromatischen Verbindung mit mindestens einem Alkylierungsmittel, das eine alkylierende aliphatische Gruppe mit mindestens sechs Kohlenstoffatomen besitzt, in Gegenwart eines Alkylierungskatalysators umfaßt, der ein Schichtmaterial umfaßt, das ein Metall-Schichtoxid und Stützen eines Oxids von zumindest einem Element enthält, das aus den Gruppen IB, IIV, IIIA, IIIB, IVA, IVB, VA, VB, VIA, VIIA und VIIIA des Periodensystems der Elemente ausgewählt ist, die die Schichten des Metalloxids trennen, wobei jede Schicht des Metalloxids Titanoxid umfaßt, das Schichtmaterial außerdem Sulfationen enthält und der Katalysator laut Elementaranalyse mindestens 0,001 Gew.-% Schwefel umfaßt.

2. Verfahren nach Anspruch 1, worin der Katalysator laut Elementaranalyse 0,1 bis 10 Gew.-% Schwefel umfaßt.

3. Verfahren nach Anspruch 1, worin der Katalysator laut Elementaranalyse 1 bis 3 Gew.-% Schwefel umfaßt.

4. Verfahren nach Anspruch 1, worin jede Schicht des Metall-Schichtoxids die allgemeine Formel aufweist

$$[M_x\square_yZ_{2 - (x + y)}O_4]^{q-}$$

worin M mindestens ein Metall der Wertigkeit n ist, worin n eine ganze Zahl zwischen 0 und 7 ist, $\square$ eine Leerstelle darstellt, Z Titan ist und worin

$$q = 4y - x(n - 4)$$
$$0 < x + y < 2$$

sind.

5. Verfahren nach Anspruch 1, worin das Führungsmaterial Trititanatschichten umfaßt.

6. Verfahren nach Anspruch 1, worin die Stützen oligomeres Siliciumdioxid umfassen.

7. Verfahren nach Anspruch 1, worin die Sulfationen hergestellt werden, indem der Katalysator mit einer wäßrigen Lösung von Schwefelsäure vorbehandelt wird.

8. Verfahren nach Anspruch 1, worin die Alkylierung bei einer Temperatur von 0 bis 500°C, einem Druck von 20 bis 25000 kPa (0,2 bis 250 Atmosphären), einer WHSV von 0,1 bis 500 und einem Molverhältnis von alkylierbarer aromatischer Verbindung zum Alkylierungsmittel von 0,1:1 bis 50:1 durchgeführt wird.

9. Verfahren nach Anspruch 1, worin die Alkylierung bei einer Temperatur von 100 bis 350°C, einem Druck von 100 bis 2500 kPa (1 bis 25 Atmosphären), einer WHSV von 0,5 bis 100 und einem Molverhältnis von alkylierbarer aromatischer Verbindung zum Alkylierungsmittel von 0,5:1 bis 5:1 durchgeführt wird.

## Revendications

1. Procédé de préparation de dérivés alkylaromatiques à longue chaîne qui comprend la mise en contact d'au moins un dérivé aromatique alkylable avec au moins un agent alkylant possédant un groupe aliphatique alkylant qui renferme au moins 6 atomes de carbone, en présence d'un catalyseur d'alkylation comprenant un matériau stratifié qui comprend un oxyde métallique stratifié et des "piliers" d'un oxyde d'au moins un élément sélectionné parmi les groupes IB, IIB, IIIA, IIIB, IVA, IVB, VA, VB, VIA, VIIA et VIIIA du tableau périodique des éléments qui séparent les couches de l'oxyde métallique, caractérisé en ce que chaque couche d'oxyde métallique comprend de l'oxyde de titane, ledit matériau stratifié comprend de plus des ions sulfate et ledit catalyseur comprend au moins 0,001% en poids de soufre, tel que mesuré par analyse élémentaire.

2. Un procédé selon la revendication 1, caractérisé en ce que le catalyseur comprend entre 0,1 et 10% en poids de soufre, tel que mesuré par analyse élémentaire.

3. Un procédé selon la revendication 1, caractérisé en ce que le catalyseur comprend entre 1 et 3% en poids de soufre, tel que mesuré par analyse élémentaire.

4. Un procédé selon la revendication 1, caractérisé en ce que chaque couche de l'oxyde métallique stratifié présente la formule générale:

$$[M_x \square_y Z_{2 - (x + y)} O_4]^{q -}$$

dans laquelle:

M représente au moins un métal de valence n, dans lequel n représente un nombre entier compris entre 0 et 7;

$\square$ représente un site vacant;

Z représente le titane; et

dans laquelle:

$$q = 4y - x(n - 4)$$
$$0 < x + Y < 2.$$

5. Un procédé selon la revendication 1, caractérisé en ce que le matériau de plomb comprend des couches de trititanate.

6. Un procédé selon la revendication 1, caractérisé en ce que les piliers comprennent du dioxyde de silicium polymérique.

7. Un procédé selon la revendication 1, caractérisé en ce que lesdits ions sulfate sont produits par prétraitement du catalyseur avec une solution aqueuse d'acide sulfurique.

8. Un procédé selon la revendication 1, caractérisé en ce que l'alkylation est mise en oeuvre à une température comprise entre 0 et 500°C, à une pression comprise entre 20 et 25 000 kPa (0,2 à 250 atmosphères), à une VSHP comprise entre 0,1 et 500 et à un rapport molaire dérivé aromatique alkylable/agent d'alkylation compris entre 0,1/1 et 50/1.

9. Un procédé selon la revendication 1, caractérisé en ce que l'alkylation est mise en oeuvre à une température comprise entre 100 et 350°C, à une pression comprise entre 100 et 2500 kPa (1 à 25 atmosphères), à une VSHP comprise entre 0,5 et 100 et à un rapport molaire dérivé aromatique alkylable/agent d'alkylation compris entre 0,5/1 et 5/1.